# EUROPEAN PATENT APPLICATION

(11) **EP 3 059 324 A1**
(43) Date of publication of application: **24.08.2016**
(21) Application number: 15382064.2
(22) Date of filing: 17.02.2015
(51) Int. Cl.: C12Q 1/68

(54) **Method for the determination of the risk of atypical fractures in patients treated with bone remodelling inhibitors**

(71) Applicant: Fundació Institut Mar d'Investigacions Mèdiques (IMIM), 08003 Barcelona (ES); Universitat de Barcelona, 08007 Barcelona (ES); Servicio Andaluz de Salud, 41071 Sevilla (ES)
(72) Inventor: Díez Pérez, Adolf, 08004 Barcelona (ES); García Giralt, Natàlia, 08004 Barcelona (ES); Nogués Solan, Francesc Xavier, 08004 Barcelona (ES); Grinberg Vaisman, Daniel Raúl, 08007 Barcelona (ES); Balcells Comas, Susana, 08007 Barcelona (ES); Quesada Gómez, Jose Manuel, 41071 Sevilla (ES)
(74) Representative: Pons

(57) **Abstract**

The present invention relates to a method for *in vitro* detecting the risk of atypical fractures in patients suffering bone diseases treated with inhibitors of bone remodelling or who are eligible for treatment with such drugs, characterized in that it comprises the detection of the mutation c.562G>T of the *GGPS1* gene. The present invention also relates to the use of the mutation c.562G>T of the *GGPS1* gene as a biomarker for determining the risk of developing atypical fractures in those individuals, a kit that comprises the elements necessary for its detection and the use of the kit for determining the risk of developing atypical fractures.

SEQUENCE LISTING

<110>  Fundacio Institut Mar D'Investigacions Mediques
       Universitat de Barcelona
       Servicio Andaluz de Salud

<120>  Method for the determination of the risk of atypical fractures in
        patients treated with bone remodelling inhibitors

<130>  EP2228.6

<160>  2

<170>  PatentIn version 3.5

<210>  1
<211>  21
<212>  DNA
<213>  Artificial Sequence

<220>
<223>  Pirmer forward

<400>  1
ccacagcatc tatggaatcc c                                                21


<210>  2
<211>  23
<212>  DNA
<213>  Artificial Sequence

<220>
<223>  Primer reverse

<400>  2
ctgttctctg gcgcaagata ttc                                              23

## Description

The present invention relates to a method for detecting the risk of atypical fractures in patients suffering bone diseases treated with inhibitors of bone remodelling or that are eligible for treatment with such drugs. Therefore, the invention could be framed in the field of Biomedicine.

### BACKGROUND ART

Atypical fractures (AF) associated with prolonged use of drugs that inhibit bone resorption are characterized by their location in the subtrochanteric region or femoral shaft. Frequently are bilateral and usually appear after a low energy trauma, equivalent to a fall from own height or less, although they also can occur spontaneously. These fractures that share some common clinical features themselves are distinct to classic osteoporotic fragility fractures (Shane E et al. JBMR 2014, 29 (1): 1-23). In order to differentiate AF from other subtrochanteric/diaphyseal femur fractures, the American Society for Bone and Mineral Research (ASBMR) has recently established the "case definition"; thus, in addition to its location in that anatomical region, should be associated with minimal trauma, must have a transverse or short oblique configuration, non-comminuted and may be associated with medial spike in complete fractures. Both complete and incomplete fractures are associated with a macroscopic periosteal reaction and thickening of the lateral cortex at the fracture site, both features indicative of stress fracture. Occasionally, cortical thickening is more generalized, affecting both cortices. Secondary features include prodromal thigh pain before fracture occurs, delayed consolidation, some associated diseases (diabetes, congestive-vitamin D deficiency, rheumatoid arthritis, hypophosphatasia) and the use of drugs (glucocorticoids, pump inhibitors protons, etc.) (Brown JP et al. Canadian Family Physician 2014, 60: 324-333).

Most AF have been associated with prolonged bisphosphonate therapy but may also occur with the use of other antiresorptive agents such as denosumab (Aspenberg P. Orthopaedica Act 2014, 85 (1): 1-1).

The absolute risk of AF associated with bisphosphonate treatment is between 2 per 100,000 patient-years at 2 years of treatment and 78 cases per 100,000 patient-years at 8 years of treatment (Brown JP et al. Canadian family physician 2014, 60: 324-333). These data lead us to think that the duration of bisphosphonate therapy positively influences the risk of triggering these fractures. But given the low absolute prevalence, it can be assumed that there are some underlying genetic causes that can trigger the onset of these fractures.

Aminobisphosphonates (alendronate, risedronate, ibandronate and zoledronic acid) are currently considered first-line treatment of osteoporotic disease. Although their anti-fracture efficacy and relative safety have been vastly demonstrated in several clinical trials, a number of adverse effects associated with prolonged use of these drugs have been described, among them the AF. Prolonged inhibition of osteoclast function and therefore of bone remodelling could cause accumulation of micro-cracks, since the bone develops an impaired capability to repair this damage. These micro-cracks would be located in areas of high mineralization, particularly in cortical bone, and thereby, the subtrochanteric and diaphyseal femur areas are especially susceptible to this type of fracture. The ASBMR has proposed several possible pathogenic mechanisms associated with atypical fracture, such as altered pattern of collagen fibers, accumulation of microfractures triggering bone fragility despite increased bone mineral density, impaired mineralization with reduced heterogeneity and an antiangiogenic effect causing a reduced vascularization.

Bisphosphonates bind to the newly formed bone with high affinity and can persist for a long time in the skeleton. Consequently, bisphosphonates bound to the bone provide a residual therapeutic action for years after the cessation of treatment. In this regard, the cumulative effect of bisphosphonates and, therefore, the prolonged reduction in bone remodelling cause an increased risk of atypical fracture from 5 to 7 years of treatment (Rosenthal Y et al. The Israel Medical Association Journal 2014, 16: 78-82).

Consequently, it is of paramount importance to know the pathophysiology of AF and their genetic determinants to improve patient management and to minimize the incidence of these fractures. The clinical translation of this discovery should be, in individuals carrying a mutation associated with this disease, to stop the treatment with inhibitors of bone remodelling if already started, or to consider prescribing different groups of drugs for treatment of bone diseases when a new treatment is indicated. So far, there are no publications of studies on genetic risk factors for AF and there is no diagnostic test that can predict the occurrence of AF associated with treatment with bone remodelling inhibitors. The high prevalence of the osteoporotic disease and the fact that antiresorptive drugs constitute the first-line of treatment, demands for a test to detect the probability of suffering a paradoxical harmful effect of these drugs, weakening the bone, and producing this kind of fracture. Therefore there is a need of a reliable method for prognosis of AF that could help to individualize the treatments by detecting the individual risk of suffering side effects by antiresorptive drugs.

### SUMMARY OF THE INVENTION

The present invention discloses an *in vitro* method for prognosis of atypical fractures in patients treated with inhibitors of bone remodelling, or that are eligible for treatment with such drugs. In the present invention, a genetic variant in the *GGPS1* gene has been identified to be associated with AF in patients treated with inhibitors of bone remodelling. This association means that individuals treated with inhibitors of bone remodelling that present the *GGPS1* variant may have an increased probability of suffering AF and, therefore, are individuals at risk.

In the present invention the term "prognosis" is the diagnosis of the risk or propensity of AF in patients who are treated with bone remodelling inhibitors, or who will be treated with such drugs.

The present invention will be of usefulness for individualized treatments in patients being treated with bone remodelling inhibitors, or patients elegible for a future treatment with such drugs by detecting the individual risk of suffering side effects of bone remodelling inhibitors, therefore of suffering AF.

Therefore, a first aspect of the present invention refers to a method to provide data for determining the risk in an individual of developing an atypical fracture comprising the detection of the mutation c.562G>T of the *GGPS1* gene, in a biological sample isolated from the individual, wherein said individual has been treated or it is going to be treated with an inhibitor of bone remodelling. From now on, we will refer to this method as the "first method of the invention".

The term "risk of developing an atypical fracture" as used herein refers to the predisposition of an individual to suffer or develop an atypical fracture.

As is well known by the expert in the field, an individual who is going to be treated with an inhibitor of bone remodelling (or that is eligible for that treatment) is the individual who presents risk of fragility fracture. The "risk of fragility fracture" is a term well known by the expert in the field and determined by conventional methods, as bone densitometry or risk scales among others (McCloskey E. Practitioner. 2013 Oct; 257(1765):19-21, 2-3.), in the field of the invention.

The *GGPS1* gene (Geranylgeranyl Diphosphate Synthase 1) is known by the expert in the field, for example with the NCBI reference sequence NM_001037277.1 (Gene ID: 9453). The gene codes for the protein geranylgeranyl pyrophosphate synthase (GGPPS; GGPPSase, GGPP synthase, geranyltranstransferase, farnesyltranstransferase, dimethylallyltranstransferase, farnesyl diphosphate synthase, or (2E,6E)-farnesyl diphosphate synthase; NP_001032354).

The mutation c.562G>T of the *GGPS1* gene (the "mutation of the invention", a replacement of a guanine by a thymine in position 562 of the coding DNA) is the allelic variant localized in chromosomal region 1:235505746. The mutation produces the change in position 188 of the protein from an aspartic residue (Asp, D) to a tyrosine (Tyr, Y), p.Asp188Tyr (D188Y).

The term "biological sample" includes, but without limitation, tissues and/or biological fluids from an individual, obtained by any method known by a subject matter expert to serve for this purpose.

A particular embodiment of the first aspect of the invention refers to a method wherein the inhibitor of bone remodelling is an aminobisphosphonate and/or anti-RANKL antibody. A more particular embodiment refers to the method wherein the aminobisphosphonate is alendronate (for example alendronic acid, Fosamax®, Fosavance®), risedronate (for example risedronic acid, Actonel®, Acrel®), ibandronate, zoledronic acid (for example Aclasta®, Zometa®), any derivative or analog of them or any combination thereof.

In the present invention "inhibitor of bone remodelling" or "antiresorptive drug" (also known as "anticatabolic drug"), as known by the expert in the field, is a drug characterized by a depression of bone resorption usually with an associated decrease in bone formation (Lems WF and Geusens P. Curr Opin Rheumatol. 2014 May;26(3):245-51).

The anti-RANKL (anti-receptor activator of nuclear factor kappa-B ligand) antibody can be for example, but not limited to, denosumab (Prolia®, Xgeva®).

A second aspect of the present invention refers to an *in vitro* method for determining the risk in an individual of developing an atypical fracture, wherein the individual has been treated or it is going to be treated with an inhibitor of bone remodelling, comprising:
a. the detection of the mutation c.562G>T of the *GGPS1* gene in a biological sample isolated from the individual;
b. the association of the presence of the mutation of step (a) with an increased risk of atypical fracture.

From now on, we will refer to this method as the "second method of the invention".

A particular embodiment of the second method of the invention comprises also the detection of any of the products of the mutation c.562G>T of the *GGPS1* gene in a biological sample isolated from the individual and the association of the presence of the mutation with an increased risk of atypical fracture.

The term "in vitro" refers to the method of the invention is done outside the body of the subject.

The second method of the invention can also comprise a step of comparison with a reference (control) sample. The term "reference sample" as understood in the present invention refers, for example, but not limited to, the samples taken from individuals who present a known genetic profile. Therefore, the reference sample can be of an individual who presents ("positive control") or who does not present the mutation ("negative control") of the present invention.

In a particular embodiment of the second aspect of the invention the inhibitor of bone remodelling is an aminobisphosphonate and/or anti-RANKL antibody. In a more particular embodiment the aminobisphosphonate is alendronate, (for example alendronic acid, Fosamax®, Fosavance®), risedronate (for example risedronic acid, Actonel®, Acrel®), ibandronate, zoledronic acid (for example Aclasta®, Zometa®), any derivative or analog of them or any combination thereof.

In a particular embodiment of the first and the second aspect of the invention the biological sample is blood, serum, plasma, tissue, oral mucosae, lymph or any other biological material obtained from the individual. The sample can be a fresh, frozen, fixed sample or a fixed sample embedded in paraffin.

The extraction of the deoxyribonucleic acid (DNA), ribonucleic acid (RNA) or protein present in the isolated biological sample can be done through protocols known in the state of the art. The isolated biological sample can be treated physically or mechanically to break the tissue or cellular structures and release the intracellular components to an aqueous or organic solution to prepare nucleic acids for analysis. Nucleic acids or proteins are extracted from the sample by known procedures by the subject matter expert.

In another particular embodiment of the first and the second aspect of the invention individual (subject) is a human (man or woman).

In another particular embodiment of the first and the second aspect of the invention the detection is performed by hybridization, sequencing, genotyping, polymerase chain reaction (PCR), restriction analysis, or by detection of the mutated protein. In a more particular embodiment, when the detection is performed by PCR and sequencing, the probes used are the sequences SEQ ID NO: 1 and SEQ ID NO: 2.

As is well understood by the expert in the field, the mutation of the invention can be detected in the gene or in the corresponding products of the gene, either the mRNA or the protein. When the detection is performed in the protein, the mutation is the p.Asp188Tyr of the GGPPS protein. Therefore, in the methods of the invention the detection of the mutation of the invention can be performed in the corresponding mRNA and/or protein.

The methods of the invention are useful to determine the genetic predisposition of an individual to suffer an atypical fracture wherein the said individual has been treated or is going to be treated with an inhibitor of bone remodelling.

A third aspect of the present invention refers to the use of the mutation c.562G>T of the *GGPS1* gene as a biomarker for determining the risk of developing AF in an individual that has been treated or it is going to be treated with an inhibitor of bone remodelling. Therefore, it is useful to determine the genetic predisposition of an individual to suffer an atypical fracture wherein the said individual has been treated or it is going to be treated with an inhibitor of bone remodelling.

The present invention also refers to the use of the corresponding mutation in the mRNA of the *GGPS1* gene (corresponding to position c.562G>T of the *GGPS1* gene) and/or the mutation p.Asp188Tyr of the GGPPS protein as biomarkers for determining the risk of developing AF in an individual that has been treated or who is going to be treated with an inhibitor of bone remodelling.

In a particular embodiment of the third aspect of the present invention the inhibitor of bone remodelling is an aminobisphosphonate and/or anti-RANKL antibody. In a more preferred embodiment the aminobisphosphonate is alendronate, (for example alendronic acid, Fosamax®, Fosavance®), risedronate (for example risedronic acid, Actonel®, Acrel®), ibandronate, zoledronic acid (for example Aclasta®, Zometa®), any derivative or analog of them or any combination thereof.

A fourth aspect of the present invention refers to a kit or a device that comprises one or more specific probes, primers or antibodies, or any combination thereof, to detect the mutation c.562G>T of the *GGPS1* gene. It also, refers to the kit or device that comprises one or more specific probes, primers or antibodies to detect the mutation of the invention in the corresponding mRNA and also to the kit that comprises specific antibodies to detect the mutation p.Asp188Tyr of the GGPPS protein. For example, the device can be an electronic device.

In a particular embodiment of the fourth aspect of the present invention the kit or device comprises the primers SEQ ID NO: 1 and SEQ ID NO: 2. In a more particular embodiment the kit consists of the primers SEQ ID NO: 1 and SEQ ID NO: 2.

The kit or device can also include at least one of the reagents selected from the list that includes: reverse transcriptase, an RNA polymerase or a fluorophore, a mixture of deoxynucleotide triphosphates (dNTPs), a mixture of nucleotide triphosphates (NTPs), deoxyribonuclease (DNase), inhibitors of the ribonuclease (RNase), dithiothreitol (DTT), inorganic pyrophosphatase (PPI) and buffers.

In addition, the present invention also relates to the kit or device of the fourth aspect wherein the probes, primers or the antibodies are preferably located on a solid support, for example, but not limited to, glass, plastic, tubes, multi-well plates, membranes, silicon supports, plastic supports, compact discs, filters, gel layers, metallic supports, any other known support, or a mixture thereof.

The specific probe or primer (non-natural occurring DNA) used in the present invention (in the methods of the invention, the kit or the device) can be modified, for example with labels suitable for its detection or chemically modified. Such labels can be all the known by the expert in the field, for example, fluorophores, radioactive isotopes, molecules for immunological identification or quenchers. Non-limiting examples of fluorescent labels that may be used within the context of the present invention include: FAM™, VIC®, HEX™, TET™, CY3, CY5.5, JOE™, ROX™, Cascade Blue®, fluorescein, phycoerythrin, Texas Red®, rhodamine, rhodamine green, rhodamine red, rhodamine 6G, 6-TAMRA, 5-TMRIA, Alexa fluor® (for example, Alexa fluor® 430, Alexa fluor® 488, Alexa fluor® 594), Bodipy®, etc. Examples of quenchers (quencher pigment or non-fluorescent quencher -NFQ- that increases the detection efficacy and the signal because it does not emit fluorescence) are, without being limited thereto, Methyl Red, ElleQuencher, Dabcyl, Dabsyl, TAMRA, etc. In the present invention, "quencher" is understood to mean a molecule that accepts energy from a fluorophore and dissipates it in the form of heat or fluorescence. Radioactive isotopes can be for example phosphorus-32 or tritium. As molecules for immunological identification, it can be used any molecule known by the expert in the field, for example digoxigenin.

The length of the specific probes or primers can be any length, preferably the length is between 10 and 50 nucleotides, preferably between 15 to 25 nucleotides, more preferably between 18 or 22 nucleotides.

A fifth aspect of the present invention refers to use of the fourth aspect of the invention for determining the risk of developing AF in an individual that has been treated or who is going to be treated with an inhibitor of bone remodelling. Therefore, it is useful to determine the genetic predisposition of an individual to suffer an atypical fracture wherein the said individual has been treated or is going to be treated with an inhibitor of bone remodelling.

In a particular embodiment of the fifth aspect of the present invention the inhibitor of bone remodelling is an aminobisphosphonate and/or anti-RANKL antibody. In a more particular embodiment the aminobisphosphonate is alendronate, (for example alendronic acid, Fosamax®, Fosavance®), risedronate (for example risedronic acid, Actonel®, Acrel®), ibandronate, zoledronic acid (for example Aclasta®, Zometa®), any derivative or analog of them or any combination thereof.

The present invention also describes a method for the treatment of an individual wherein the individual has been treated or is going to be treated with an inhibitor of bone remodelling, preferably wherein the inhibitor of bone remodelling is an aminobisphosphonate and/or anti-RANKL antibody, more preferably wherein the aminobisphosphonate is alendronate, (for example alendronic acid, Fosamax®, Fosavance®), risedronate (for example risedronic acid, Actonel®, Acrel®), ibandronate, zoledronic acid (for example Aclasta®, Zometa®), any derivative or analog of them or any combination thereof; which comprises modification of the treatment or the avoidance of the use of the aminobisphosphonate in case the mutation c.562G>T of the *GGPS1* gene (corresponding to the mutation p.Asp188Tyr of the GGPPS protein) is present. The "modification of the treatment" refers to providing the patient with an alternative treatment that would not include the inhibition of bone remodelling, such as the administration of teriparatide (Forteo®, Forsteo®), or the combination of teriparatide with another treatment that would not include the inhibition of bone remodelling.

The terms "amino acid sequence" or "protein" are used here interchangeably and refer to a polymeric form of amino acids of any length, which may or may not be, chemically or biochemically modified.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### BRIEF DESCRIPTION OF THE DRAWINGS

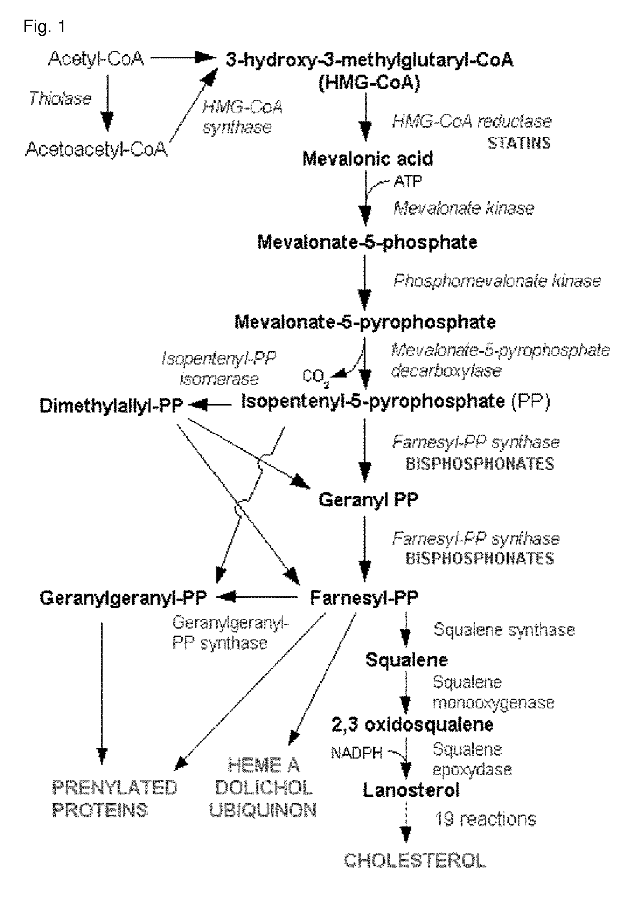

**Fig. 1****.** Bisphosphonates act at two metabolic steps related to Geranylgeranyl pyrophosphate (GGPP) (Ganapathy N et al., Bisphosphonates: An update. Dental Science - Review article (2012), vol.4 (6), pg. 410-413).

### EXAMPLES

### Example 1: the mutation of the invention is useful in the prognosis of AF in patients treated with inhibitors of bone remodelling or who are eligible for treatment with such drugs.

DNA of three sisters, suffering AF associated to long-term bisphosphonate use, was extracted from peripheral blood with the Wizard Genomic DNA Purification Kit (Promega). These DNAs were used for whole exome sequencing. The library preparation for capturing of selected DNA regions (SureSelect XT Human All Exon; cat: 5190-6208; Agilent Technologies) was performed according to Agilent's SureSelect protocol for Illumina paired-end sequencing. In brief, 3.0µg of genomic DNA was sheared on a Covaris™ E220 instrument. The fragment size (150-300 base pairs) (bp) and quantity were confirmed with the Agilent 2100 Bioanalyzer 7500 chip. The fragmented DNA was end-repaired, adenylated and ligated to Agilent indexing-specific paired-end adaptors. The DNA with adaptor-modified ends was PCR amplified (6 cycles, Herculase II fusion DNA polymerase from Agilent) with SureSelect Primer and SureSelect Pre-capture Reverse PCR primers (SureSelect XT Human All Exon), quality controlled on the DNA 7500 assay for the library size range of 250 to 450 bp and hybridized for 24h at 65°C (Applied Biosystems 2720 Thermal Cycler). The hybridization mix was washed in the presence of magnetic beads (Dynabeads MyOne Streptavidin T1, Life Technologies) and the eluate was PCR amplified (16 cycles) in order to add the index tags using SureSelectXT Indexes for Illumina. The final library size and concentration was determined on Agilent 2100 Bioanalyzer 7500 chip and sequenced on an Illumina HiSeq 2000 platform with paired end run of 2x76bp following the manufacturer's protocol. Images from the instrument were processed using the manufacturer's software to generate FASTQ sequence files. The bioinformatic analysis was performed in the Bioinformatics Platform for Rare Diseases CIBERER (CIBERER Bier) in Valencia. The data in format FASTQ of the sequenced fragments were aligned with the Burrows-Wheeler Aligner software free (Burrows M and Wheeler DJ. Technical report 124. Palo Alto, CA: Digital Equipment Corporation; 1994.) using the build GRCh37 (hg19) of reference human genome, so each fragment was situated in its genomic position.

Genetic variants found in the three sisters were filtered according to three premises: a) non-synonymous change, b) not previously described or with a Minor Allele Frequency < 0.005 and c) not present in exomes of individuals drawn from the general population (n=8).

Filtered mutations were validated by polymerase chain reaction (PCR) and automatic DNA sequencing by the Sanger method (automatic Sanger sequencing). PCR is used to amplify a specific region of a DNA strand (the DNA target) across several orders of magnitude, generating thousands to millions of copies of a particular DNA sequence. Automatic Sanger sequencing is used to determine the precise primary sequence (nucleotide sequence) of a specific region of a DNA strand. Primary sequences were aligned to the reference human genome sequence to confirm that the genetic variants detected by the Illumina procedure were true variants and not sequencing errors (validation). One mutation in the *GGPS1* gene (c.562G>T) in the genomic position 1:235505746 was validated using the primer forward 5'-CCACAGCATCTATGGAATCCC-3' (SEQ ID NO: 1) and the primer reverse 5'-CTGTTCTCTGGCGCAAGATATTC-3' (SEQ ID NO: 2).

Moreover, this mutation was not detected in 50 DNA samples from population controls and in 3 samples from women without atypical fracture and long-term bisphosphonate use. The *in silico* functional study of the protein GGPPS was performed using The Universal Protein Resource (UniProt), RCSB Protein Data Bank (PDB) and Pfam.

The mutation described in the present invention p.Asp188Tyr (D188Y) in the GGPPS protein is located in an α-helix and specifically, in the residue which binds one of the three magnesium ions needed for the enzyme catalytic activity. The mutation elicits the change of an acidic amino acid (aspartate) to an aromatic amino acid without charge (tyrosine) and which usually occupies large spaces. This change is likely to disrupt both the magnesium union and the α-helix secondary structure. The predictors Sift and Poliphen both predict that this variant is highly deleterious.This deleterious effect and the fact that this mutation has been detected in three sisters with atypical fracture after long-term bisphosphonate use (and not in population controls) strongly suggest that this genetic variant plays a causal role of in the occurrence of AF. Therefore the individuals carrying the mutation, when exposed to inhibitors of remodelling, will be at increased risk of suffering AF, either if already receiving these drugs or when considering initiating treatment with these drugs. Therefore, the detection of this mutation is useful to patients in treatment and also for patients elegible for the mentioned treatment. Interestingly, geranylgeranyl diphosphate synthase (GGPPS) is involved in the mevalonate pathway. The GGPPS enzyme catalyzes the synthesis of GGPP from farnesyl diphosphate and isopentenyl diphosphate. The GGPP is an important precursor of carotenoids and geranylgeranylated proteins such as those belonging to the Rho family (small GTPases) essential for cellular activities including morphology, motility, and proliferation. Bisphosphonates act by inhibiting farnesyl pyrophosphate synthase (FPPS), thereby preventing prenylation and activation of small GTPases that are essential for the activity and survival of osteoclasts (the bone resorbing cells). The FPPS enzyme, also involved in the mevalonate pathway, provides the substrate for GGPPS (FIG. 1). Moreover, a genetic variant (-8188A ins/del) in the promoter region of the *GGPS1* gene has been associated with femoral neck BMD response to bisphosphonate therapy (Choi HJ et al. Yonsei Med J. 2010 Mar;51(2):231-8), suggesting that variants of this gene may interact with the action of bisphosphonates. All these evidences support the causality of the mutation D188Y in the risk of AF after long-term bisphosphonate use.

## Claims

1. A method to provide data for determining the risk in an individual of developing an atypical fracture comprising the detection of the mutation c.562G>T of the gene *GGPS1,* in a biological sample isolated from said individual, wherein the individual has been treated or is going to be treated with an inhibitor of bone remodelling.

2. The method according to claim 1, wherein the inhibitor of bone remodelling is an aminobisphosphonate and/or anti-RANKL antibody, preferably the aminobisphosphonate is alendronate, risedronate, ibandronate, zoledronic acid, or any derivative or analog of them; or any combination thereof.

3. An *in vitro* method for determining the risk in an individual of developing an atypical fracture, wherein the individual has been treated or it is going to be treated with an inhibitor of bone remodelling, comprising:
a. the detection of the mutation c.562G>T of the gene *GGPS1* in a biological sample isolated from the individual;
b. the association of the presence of the mutation of step (a) with an increased risk of atypical fracture.

4. The method according to claim 3 wherein the inhibitor of bone remodelling is an aminobisphosphonate and/or anti-RANKL antibody; preferably the aminobisphosphonate is alendronate, risedronate, ibandronate, zoledronic acid or any derivative or analog of them; or any combination thereof.

5. The method according to any one of claims 1 to 4 wherein the biological sample is blood, serum, plasma, tissue, oral mucosae or lymph.

6. The method according to any one of claims 1 to 5 wherein the individual is a human.

7. The method according to any one of claims 1 to 6 wherein the detection is performed by hybridization, sequencing, genotyping, polymerase chain reaction (PCR), restriction analysis, or by detection of the mutated protein.

8. The method according to claim 7 wherein when the detection is performed by PCR the primers used are the sequences SEQ ID NO: 1 and SEQ ID NO: 2.

9. The use of the mutation c.562G>T of the *GGPS1* gene as a biomarker for determining the risk of developing atypical fractures in an individual who has been treated or who is going to be treated with an inhibitor of bone remodelling.

10. The use according to claim 9 wherein the inhibitor of bone remodelling is an aminobisphosphonate and/or anti-RANKL antibody, preferably the aminobisphosphonate is alendronate, risedronate, ibandronate, zoledronic acid or any derivative or analog of them; or any combination thereof.

11. A kit or device that comprises one or more specific probes, primers or antibodies to detect the mutation c.562G>T of the *GGPS1* gene, preferably wherein the probes or primers are modified.

12. The kit or device according to claim 11 wherein the kit or device comprises the primers SEQ ID NO: 1 and SEQ ID NO: 2, preferably wherein the primers are modified.

13. A use of the kit according to any one of claims 11 or 12 for determining the risk of developing atypical fractures in an individual who has been treated or who is going to be treated with an inhibitor of bone remodelling.

14. The use according to claim 13 wherein the inhibitor of bone remodelling is an aminobisphosphonate and/or anti-RANKL antibody; preferably the aminobisphosphonate is alendronate, risedronate, ibandronate, zoledronic acid or any derivative or analog of them; or any combination thereof.
